# EUROPEAN PATENT SPECIFICATION

(11) **EP 1 620 157 B1**
(45) Date of publication and mention of the grant of the patent: **16.11.2011**
(21) Application number: 04749610.4
(22) Date of filing: 01.04.2004
(51) Int. Cl.: A61M 25/00

(54) **GUIDE WIRE HAVING BENDING SEGMENT**
FÜHRUNGSDRAHT MIT EINEM BIEGESEGMENT
FIL DE GUIDAGE A SEGMENT FLEXIBLE

(30) Priority: 03.04.2003 US 406020; 03.04.2003 GB 0307715; 08.04.2003 US 409270; 05.12.2003 US 729754
(43) Date of publication of application: 01.02.2006
(73) Proprietor: ETHICON ENDO-SURGERY, INC., Cincinnati, OH 45242 (US); UCL Business PLC, London W1T 4TP (GB)
(72) Inventor: BAKOS, Gregory, J., Mason, OH 45040 (US); GEE, Kevin, K., Scituate, MA 02066 (US); TIERNEY, Scott, J., Taunton, MA 02780 (US); SWAIN, Christopher, Paul, London NW3 1TN (GB); LONG, Gary, L., Cincinnati, Ohio 45277 (US)
(74) Representative: Boon, Graham Anthony
(86) International application number: PCT/US2004/009966
(87) International publication number: WO 2004/089455

(56) References cited:
- US-A- 5 113 872
- US-A- 5 363 847
- US-A- 5 891 055
- US-A- 6 106 488
- US-A1- 2002 010 426
- US-A1- 2002 161 393

## Description

**Field of the Invention**

The present invention is related generally to a guide wire structure. In one embodiment, the invention is directed to a guide wire structure which can be inserted into an interior space within a human or animal body, such as the gastrointestinal (GI) tract of a human patient.

**Background of the Invention**

A physician typically accesses and visualizes tissue within a patient's gastrointestinal (GI) tract with a long, flexible endoscope. For the upper GI, a physician may insert a gastroscope into the sedated patient's mouth to examine and treat tissue in the esophagus, stomach, and proximal duodenum. For the lower GI, a physician may insert a colonoscope through the sedated patient's anus to examine the rectum and colon. Some endoscopes have a working channel, typically about 2.5-3.5mm in diameter, extending from a port in the handpiece to the distal top of the flexible shaft. A physician may insert medical instruments into the working channel to help diagnose or treat tissues within the patient. Physicians commonly take tissue biopsies from the mucosal lining of the GI tract using a flexible, biopsy forceps through the working channel of the endoscope.

Insertion of a flexible endoscope, especially into the colon, can be very time-consuming and uncomfortable procedure for the patient, even when sedated with drugs. A physician often needs several minutes to push a flexible endoscope through the convoluted sigmoid, descending, transverse, and ascending portions of the colon. The physician may diagnose and/or treat tissues within the colon either during insertion or removal of the endoscope. The flexible endoscope may "loop" within the colon, such as at the sigmoid colon or at the splenic flexure of the colon, so that it becomes difficult to further advance the endoscope along the colon. When a loop is formed, the force exerted to push the scope stretches the mesentery and causes pain for the patient. Depending on the anatomy of the patient and the skill of the physician in manipulating the flexible endoscope, some portions of the colon may be unexamined, thus increasing the risk of undiagnosed disease.

Guide wires have been used to aid the introduction of catheters and other instruments into many sites in the human body. Many medical applications and specific designs of guide wires have been for cardiovascular use. There are, however, specific challenges relates to the use of guide wires in the GI tract, as opposed to the vascular system. Thus, the bowel is more tortuous, softer and generally of larger diameter. Furthermore, in the case of the small intestine and the colon, these are longer than most arteries or veins.

By way of further prior art attention is directed to US Patent Application Publication No. 2002/0161393.

**Summary of the Invention**

In one embodiment, the present invention provides a guide wire structure for use with a medical device for insertion into a body lumen, such as the GI tract. The guide wire structure comprises a continuous, unitary wire comprising at least a first segment, a second segment, and a third segment disposed intermediate the first and second segments. The third segment has a bending moment of inertia less than a bending moment of inertia of the first segment and less than a bending moment of inertia of the second segment. The third segment can provide a flexible hinge for bending of the unitary wire. A first sleeve encircles the first segment and a second sleeve encircles the second segment, wherein the first and second sleeves are visually distinguishable. By the phrase "continuous, unitary wire" it is meant the portion of the wire associated with the third segment and adjacent portions of the first and second segments do not include any joints, junctures, or other connections (such as for instance welds, braze joints, or solder joints), although the ends of the wire may include a joint or connection for connecting the wire to a handle or for other purposes. In one embodiment the wire is formed of a single material, such as a superelastic material. One suitable material from which the wire may be formed is Nitinol.

In one embodiment, the third segment has a cross-sectional area less than the cross sectional areas of the first segment and the second segment. The reduced cross-sectonal area of the third segment can be formed by grinding the outer diameter of the wire to form a reduced cross-sectional area third segment between first and second segments having a generally constant cross sectional area. The wire can have a circular cross-section, or alternatively, non-circular cross-sections. A generally conical transistion segment can extend from each end of the third segment to connect the third segment to the first and second segments.

**Brief Description of the Drawings**

The invention is described further below with reference to the accompanying drawings, in which:

Figure 1a shows an embodiment of guide wire structure as disclosed in US Patent 7288074.

Figure 1b shows the structure of Figure 1a when one of its guide wires is advanced rightwardly and the other is held steady;

Figure 1c shows the structure of Figure 1a after further righthand advance of one of the guide wires;

Figure 2 shows an example of a pattern of markings which may be provided on the guide wires to indicate their relative position to a physician;

Figure 3a to 3c show a guide wire structure advancing into the colon;

Figure 4 shows diagrammatically a handle for use in controlling movement of guide wires;

Figures 5a and 5b show successive stages in the use of a guide wire structure in conjunction with a bias tube;

Figures 6a and 6b show successive stages in the use of a cutting catheter to sever the junction between two guide wires;

Figure 7 shows two guide wire structures arranged in parallel;

Figures 8a to 8c illustrate diagrammatically the use of a guide wire structure which has a pivotal junction portion;

Figure 9 shows another guide wire structure described in US Parent 7288074.

Figure 10 illustrates an embodiment of the present invention in which a guidewire cross section is varied along its length to have a reduced cross section at a location spaced from the ends of the wire, such as at or close to the midpoint of length of the guide wire.

Figure 11 shows the guide wire of Figure 10 bent into a generally U-shaped configuration for passage into a lumen such as the GI tract.

Figures 12a, b, and c show alternative embodiments in which different cross-sections are employed.

Figure 13 illustrates an embodiment of the guide wire of the present invention being advanced from the distal end of a medical device to form a loop forward of the distal end of the medical device.

**Detailed Description**

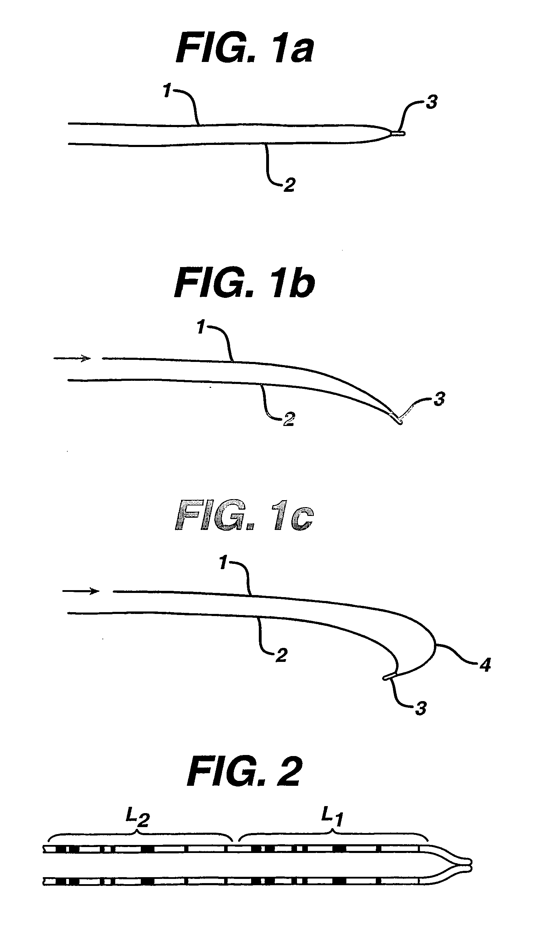

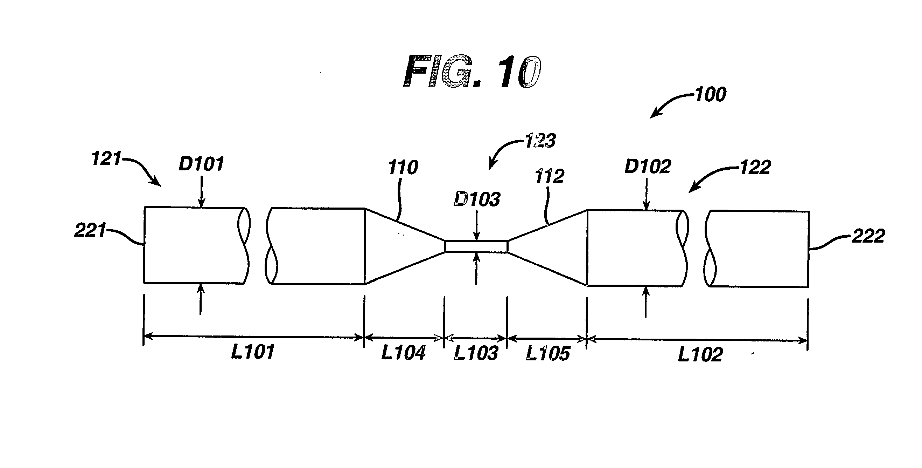

Figures 1-9 illustrate guide wire structures disclosed in US Patent 7288074. Figures 10-13 illustrate a guide wire structure according to the present invention.

The structure of Figure 1a comprises a first guide wire 1 and a second guide wire 2, the wires 1 and 2 being connected to one another by a junction 3 formed at the leading ends of the wires 1 and 2. Although the junction 3 is shown as being at the leading ends, it could alternatively be adjacent the leading ends. The length of the junction need be no more than is necessary to hold the leading ends securely together side by side. Depending on the nature of the junction, a length of as little as 5-10 mm may be sufficient, though a greater length may sometimes be preferable.

The guide wires 2 and 3 can be made of the materials conventionally used for guide wires, for example straight stainless steel wire, coiled stainless steel wire, glass fiber, a plastics material, or nitinol. Conveniently, a guide wire has a floppy tip, i.e. a leading end portion, typically 4-5 cm in length, of greater flexibility than the remainder of the guide wire, in order to reduce the risk of the leading end of the guide wire causing damage to the wall of the lumen through which it is passing. Where two such conventional guide wires are joined together to produce the guide wire structure of Figure 1, it can be these floppy tips, or parts thereof, which are joined together. The length of the junction can be less than the length of the floppy tips, so that some length of floppy material remains which is unaffected by the junction.

The whole or part of each of the guide wires may be coated to reduce its coefficient of friction, as is done with conventional guide wires. For example, guide wires can be coated with a low friction material such as silicone, or with a hydrophilic material which becomes slippery in use in a patient, or with both a low friction material such as silicone and hydrophilic material applied over the low friction material.

The junction 3 can be formed in any desired manner, provided the resulting leading end of the guide wire structure is not such as to damage the wall of the GI tract or other body lumen, nor cause undue pain when in contact therewith. For example, the junction can be made by gluing or welding the leading end portions together and then covering those portions with heat shrink tubing. Alternatively, the end portions could be held together by having a metal band crimped on to them, optionally enclosed by a cover made of a softer material.

It is not essential for all the guide wires, or both the guide wires, as the case may be, to be of material which would normally be regarded as guide wire material. For example, in the case of a guide wire structure consisting of just two guide wires, one of the guide wires may be made of a thread, which is joined to the other guide wire by being tied to it.

Another possibility would be to start with a single guide wire of twice the required length and fold it sharply back on itself, for example by crimping the folded wire adjacent the fold, so that it became, in effect, a pair of guide wires joined at the fold. A guide wire structure having an even number n of guide wires greater than two could be formed by folding half that number of guide wires.

The principle of operation of the guide wire structure can be seen by comparing Figures 1b and 1c with Figure 1a. Figure 1b shows the result of advancing the guide wire 1 rightwardly, as indicated by the arrow, whilst holding the guide wire 2 still. As indicated in Figure 1b, this causes the distal region of the guide wire structure to curve in a direction so that the advanced guide wire 1 is on the outside of the curve and the still guide wire 2 is on the inside of the curve. Continued advancement of guide wire 1 beyond the position illustrated in Figure 1b, whilst continuing to hold guide wire 2 steady, results in the formation of a loop in an end region of guide wire 1. This is illustrated in Figure 1c, where the loop is denoted by reference numeral 4.

To enable the physician to easily advance one of the guide wires while keeping the other still, the guide wires can be received, at their ends remote from the junction 3, in a handle which can be moved up and down the guide wires as they are advanced and retracted. The handle should allow precise regulation of the relative lengths of the two guide wires. It should also allow the introduction of the various catheters, imagers and other accessories, discussed in more detail below, giving accurate information on their relationship to the junction 3. The handle may be provided with a reversible motor drive which enables both guide wires to be driven. The motor drive itself may provide data to enable the user to monitor the lengths of the guide wires which have been fed forward.

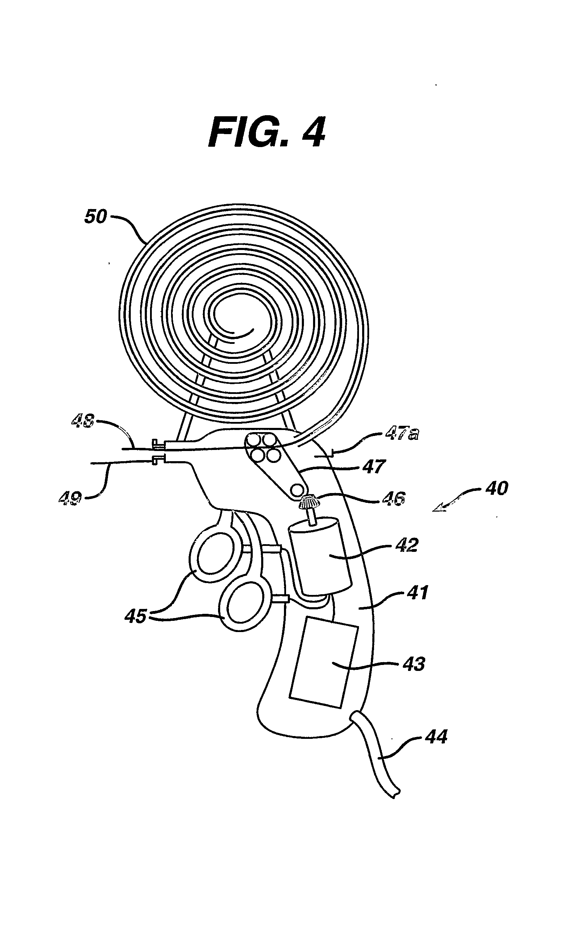

An example of a handle is illustrated in Figure 4. The illustrated handle 40 comprises a pistol grip 41 within which is mounted a pair of electric motors 42 (of which one is shown) powered either by a battery 43 or a mains supply 44. The motors are controlled by respective finger controls 45, one for each motor, each control having forward, reverse and stop positions. Each motor provides drive, via a respective gear, shown diagrammatically at 46, to a respective belt or chain drive 47, each of which propels a respective guide wire 48 forwardly (or backwardly). A switch 47a is provided to cause the driving belts or chains to move away from the wires, to allow the wires to be released, for example at the conclusion of a procedure. A lock mechanism 49 is provided to attach the handle 40 to a catheter or to an accepting channel of an endoscope, through which the guide wire is to be driven. The guide wires are stored in a coiled plastics tube 50, either with both wires side by side in a single tube or each in its own tube. This has the benefit of keeping the guide wires clean, and avoiding the risk of their trailing on to the floor. Under some conditions this storage facility may be omitted.

The combined effect of the forms of behaviour illustrated in Figures 1b and 1c enables the guide wire structure to perform in a highly advantageous manner. Thus, causing the structure to become curved, as shown in Figure 1b, enables the physician to steer the leading end of the structure round bends in the lumen through which the structure is being advanced. The ability to form a loop, as illustrated in Figure 1c, enables the guide wire structure to adopt as configuration in which it can be safely advanced along the lumen, without undue discomfort for the patient.

Furthermore, the presence of a loop at the leading end of the structure rather than the tip of a single wire, makes the structure more likely to follow the main course of the lumen, and less likely to inadvertently enter branches off it. Thus, in the case of the gut, there will be a much reduced tendency to enter, for example, diverticulae or the orifice of the appendix. However, the fact that the loop is not permanently present, and can be eliminated by putting the structure into the configuration shown in Figure 1a, means that the structure can easily, and without damage to itself, be passed along a very narrow passageway. It can therefore be passed, for example, along a channel of an endoscope or down a catheter, as is described further below. Also, when the guide wire structure is not in an endoscope or catheter, but is advancing directly along a patient lumen, it is not always desirable to do so with a loop at the front (for example if it has to pass through a small opening). Under such circumstances the guide wire structure is allowed to revert to the straight form shown in Figure 1a with both guidewires being advanced aligned and in unison.

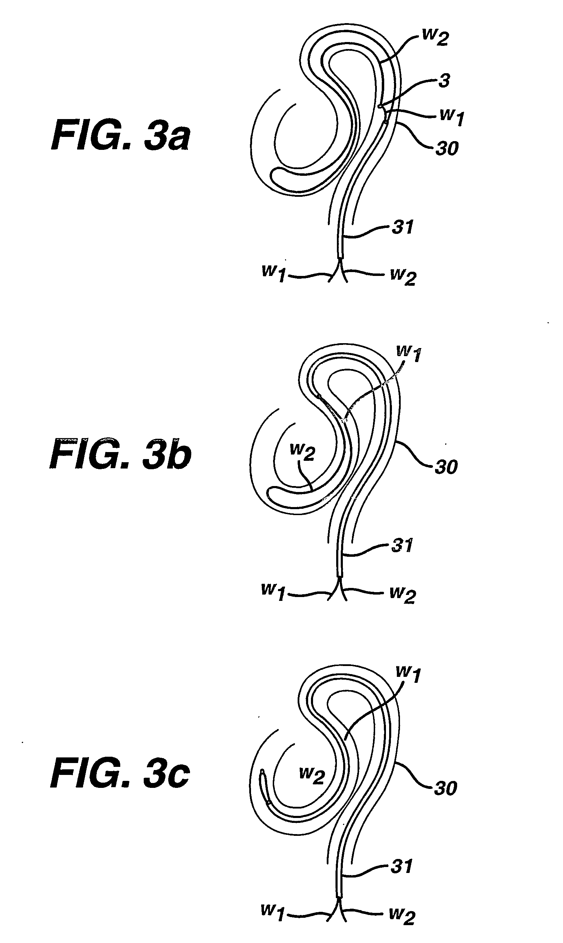

Figures 3a to 3c show diagrammatically, and by way of example, successive stages in advancing the guide wire structure along a colon 30. It is shown being introduced in conjunction with a catheter 31 within which the whole guide wire structure is slidably received. The individual guide wires are denoted as W₁ and w₂. Advancement takes place by alternately:

(a) pushing one wire forward while holding the other still; and

(b) pushing the catheter forwards as far as the position shows in Figure 3c, or even somewhat further.

It is desirable in endoscopic procedures to avoid, or at least reduce, the use of X-ray imaging to monitor what is taking place. With this in mind, the guide wires are preferably each provided with a pattern of markings, distributed along their length, to indicate how far each individual guide wire has been inserted. One such pattern in shown in Figure 2. As shown there, a pattern of markings in a given colour, and similar in nature to a bar code, is spaced along a first length (L₁), and then repeated along successive lengths (of which only L₂ is shown) each time in a different colour. Each of the lengths could conveniently be of the order of 10cm. This provides a method by which the physician can easily see which of the guide wires is the further advanced, and by how much, and enable him, for example, to make the inserted lengths equal and thus eliminate any curve (Figure 1b) or loop (Figure 1c). Of course, many other patterns of marking, for example numerals or letters, could be used instead of that illustrated, which is given only as an example.

Additionally, or instead, the guide wire structure can be provided with other forms of position indication. It is known to provide a conventional guide wire with a series of miniature electrically conductive coils which surrounded the guide wire and are spaced along its length, the coils being connected to a source of electrical current, whereby each coil becomes a miniature electromagnet. Such coils can be provided on the guide wires used to form the guide wire structure shown. A sensing device outside the patient is used to detect the position of the coils within the patient, and thereby determine the location of the guide wires.

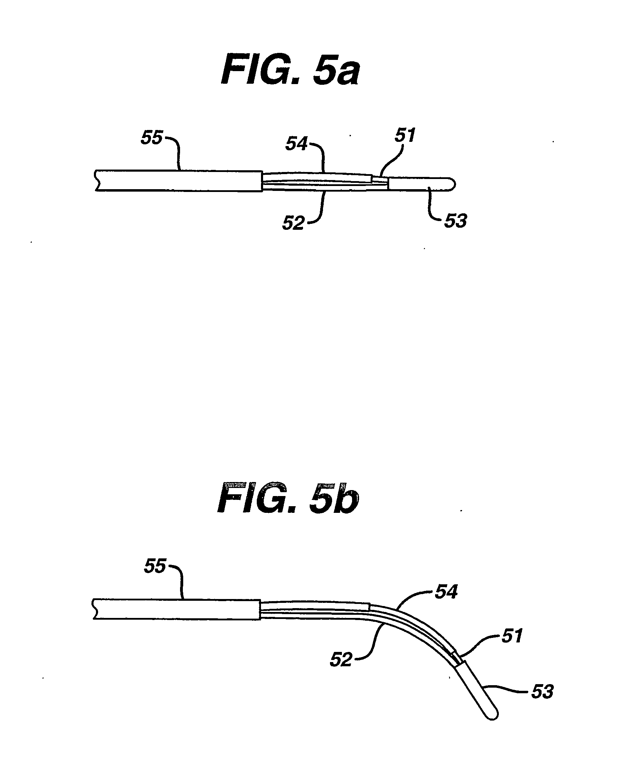

The path of the guide wire structure can be influenced by the use of a catheter, which can be passed over one or both of the two guide wires, when there are precisely two, or over one, some, or all of the guide wires, when there are more than two. In one embodiment the catheter has a curved tip, which allows the application of torque to bias the forward motion of the guide wire (or wires) over which it passes in any given direction. The use of a catheter in this way is illustrated in Figures 5a and 5b. Figures 5a and 5b show a pair of guide wires 51 and 52 joined at a junction 53. Guide wire 51 is received within a catheter 54, referred to herein as a bias tube, the leading end portion of which is so formed as to have a curvature in it. The guide wire 51 with the bias tube, and the guide wire 52, are both received within an outer catheter 55. The ends of the catheters 51 and 52 remote from their tips emerge from the catheter 55 to allow them to selectively advance and retract. The end of the bias tube 54 remote from the curved end thereof emerges from the outer catheter 55 at the user's end. As can be seen by comparing the state shown in Figure 5a with the subsequent state shown in Figure 5b, in advancing both the guide wires, but advancing guide wire 51 more than guide wire 52, the bias tube helps to ensure that the combined guide wire structure curves in the desired direction. If it were desired to cause the structure to advance in some other direction, this could be achieved by twisting the catheter 55 about its longitudinal axis, thus altering the positions of the guide wires relative to the lumen in which they are being advanced.

The purpose of the guide wire is, as its name indicates, to act as a guide for some other element. Accordingly, when the guide wire structure is in place some other element is then passed over it, or otherwise pushed or advanced along the guide wire.

As in the case of a catheter used to influence the path of a guide wire structure during passage of the guide wire structure along a lumen, a catheter introduced subsequently can pass over one or both of the guide wires, when there are precisely two, or over one, some, or all of the guide wires, when there are more than two. When the catheter is passed over both, or all, the guide wires, as the case may be, the leading end of the catheter will be free to pass beyond the leading end of the guide wire structure once it reaches that point. If the catheter is not passed over both, or all, the guide wires, for example if it is passed over only one of two interconnected guide wires, the leading end of the catheter will normally be unable to pass beyond the connection between the guide wires. That may be desirable, for the purpose of ensuring that the leading end of the catheter can be brought to a position previously defined by the leading end of the guide wire structure. It also has the result, however, that if the guide wire structure is withdrawn, the catheter must be withdrawn with it.

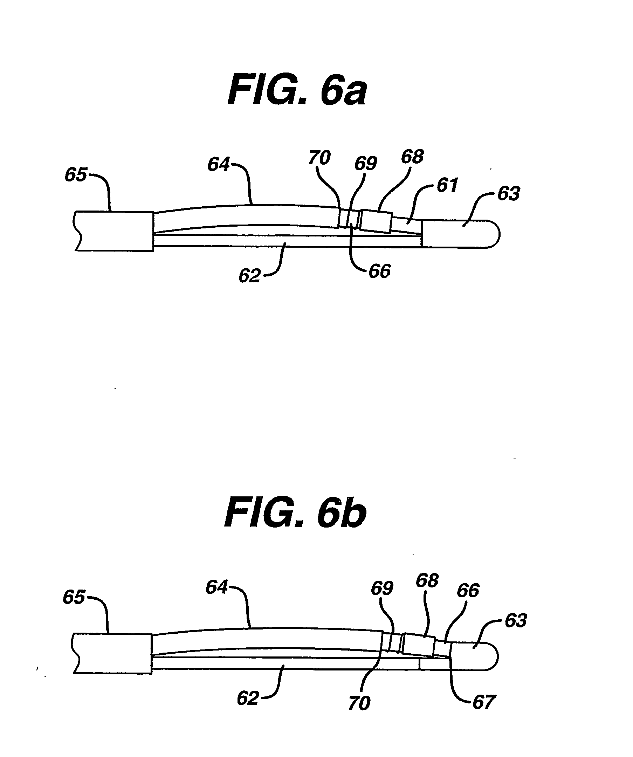

If it is desired to enable the leading end of the catheter to pass beyond the end of the guide wire over which it is traveling, or to enable the catheter to remain in position after the guide wire has been withdrawn, this can be achieved by providing the leading end of the catheter with a cutting device. The use of such a catheter is illustrated in Figures 6a and 6b. Figures 6a and 6b show guide wires 61 and 62 connected by a junction 63 and extending within an outer catheter 65. A cutting catheter 64 surrounds one of the guide wires, in this case the guide wire 61. The catheter 64 has a cutting tip (not visible in Figure 6a) which, when the catheter 64 is advanced over the guide wire 61, severs the junction 63. Figure 6b shows the severing operation partly completed.

The cutting catheter comprises a cylindrical cutting member 66 with a circular cutting edge 67 (visible in Figure 6b but not in Figure 6a) formed at its leading end. When not in use the cutting edge is shielded by a generally cylindrical sheath 68 which is biased to a forward protecting position by a compression spring 69 located between the rearward end of the sheath 68 and a stop 70 fixed to the end of the catheter. When the cutting catheter is pushed forwards, against the force of the spring 69, as it is in Figure 6b, the cutting edge 67 emerges from the sheath 68 and severs the junction 63. As soon as severing is completed the spring automatically causes the sheath 68 to move forwards, covering the cutting edge 67 and preventing it from harming the patient.

Once a sufficiently large guide wire loop has been formed in, say, the gut, it becomes possible to pull the gut backwards to some extent, using the friction between the loop and the wall of the gut. To do this, both guide wires are pulled backwards in synchronism. This provides a means for straightening the gut, and this in turn makes it easier to advance the guide wire structure further or, indeed, to advance other structures (e.g. endoscopes), and reduces the pain of the procedure, which is mainly caused by stretching nerve endings in the mesentery.

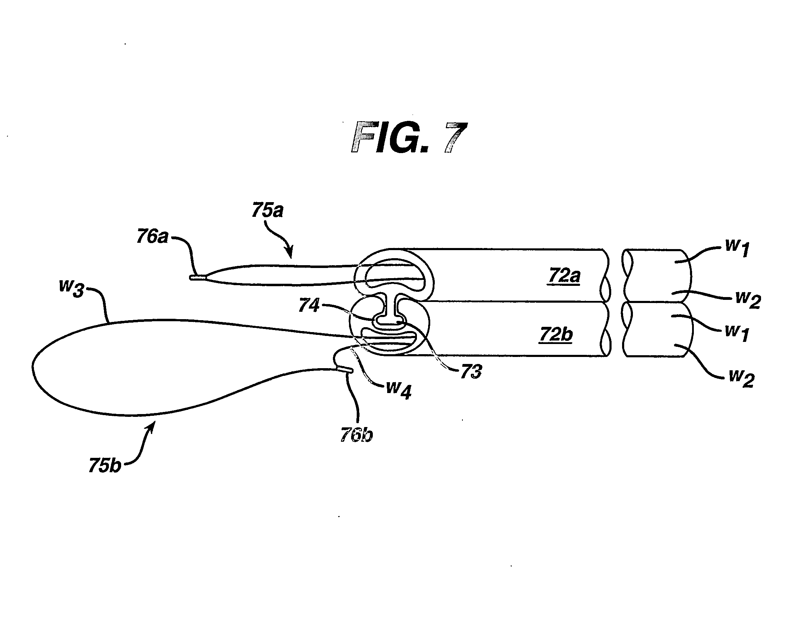

The above described concept of using a guide wire loop to straighten a passageway, e.g. the gut, can employ two guide wire structures operating in parallel. An example of such an embodiment is shown in Figure 7. This comprises two parallel catheters 72a and 72b, which are preferably connected together side by side in such a way as to allow each to move longitudinally with respect to the other. In the illustrated embodiment the connection is provided by a T-shaped stud 73 formed on catheter 72a which is slidable in a correspondingly shaped passageway 74 formed in catheter 72b and running longitudinally along it. A single stud may be provided, or a plurality of studs spaced along the length of catheter 72a, or there may be a continuous stud running along all or part of the length of catheter 72a. Catheter 72a receives a first guide wire structure 75a, comprising a pair of wires W₁ and W₂ joined at a junction 76a. Catheter 72b receives a guide wire structure 75b, comprising a pair of wires W₃ and W₄ joined at a junction 76b.

The embodiment shown in Figure 7 can be used in a procedure not claimed which employs the following steps:

1. Push the combination of catheters 72a and 72b into an appropriate orifice, e.g. the anus in the case of the colon, as far as they will go.

2. Advance wire W₃ as far as the loop which it forms is able to travel (this is substantially the configuration shown in Figure 7).

3. Pull back on both catheters so that the loop in guide wire structure 75b straightens the gut.

4. Advance guide wire structure 75a in its unlooped form, i.e. wires W₁ and W₂, through the catheter 72a as far as it will go (which should be past the loop in guide wire structure 75b).

5. Advance catheter 72a over W₁ and W₂ so that it is ahead of catheter 72b, while catheter 72a, and the loop extending from the catheter, hold the gut in position.

6. Advance guide wire W₁ or guide wire W₂ so that a loop is formed in guide wire structure 75a and advances in the gut.

7. Withdraw whichever of wires W₃ and W₄ is the more forward of the two, so as to eliminate the loop in guide wire structure 75b.

8. Advance catheter 72b so that it catches up with catheter 72a.

The above cycle is then repeated until the desired degree of advancement has been achieved.

A similar cycle of steps can be achieved by a modified form of the embodiment of Figure 7, in which one or each of the two catheters 72a and 72b is replaced by a suction catheter. A suction catheter can be used to effect the above described straightening of the gut by pulling back on it while suction is being applied. The suction is only applied during the straightening step. Yet another modification is to replace one of the guide wire structures by a soft balloon, which can be inflated to engage the gut wall, and then pulled back to straighten the gut.

Many different devices can be passed over the guide wire structure, and some examples will now be given.

(a) A small imager (for example a CCD or CMOS chip) on a catheter could be passed along the guide wire or guide wires to the tip. This could optionally be propelled along the guide wire by a water jet or some other means of tip propulsion to reduce the force that has to be exerted outside the patient. A source of white or coloured light could be also introduced by the same means. This source could be in the form of light emitting diodes or could use fibre-optics. One of the wires could be optionally formed out of a fiberoptic bundle. It would be easier to take the optical signal through a light-weight insulated wire which could be incorporated into the guide wire or via a separate wire in a catheter. The imager could then convert the optical information to radiowaves or microwaves, to send the information to an aerial attached to, or adjacent to, the exterior of the patient.

(b) A separate soft catheter could be run over the guide wire to the tip and this could be used to introduce air from a controlled pump to inflate the viscus. Water for rinsing purposes could be passed through this catheter or through some other from a water pump.

(c) A catheter could be passed over one of the guide wires, which would provide a channel through which biopsies could be performed. This is preferably done after the imager referred to in (a) above has been placed in position, so that the imager can be used to view the biopsy procedure. This catheter might have tip angulation properties.

(d) A double lumen catheter could be passed over the double wire, which might allow the introduction of another wire of greater stiffness or with a curled tip to allow the movement of the device in a desired direction.

Once the guide wire, and the imager referred to in (a) above, have reached the desired location, an overtube could be passed, for example to the cecum. The guide wire and the imager could then be withdrawn and a conventional endoscope could be passed through the overtube to deliver therapy, for example removing a polyp or cancer.

A conventional endoscope could be introduced into a body lumen by passing it over the guide wire structure. However, a conventional endoscope may be too stiff for this to be possible, and the guide wire structure offers the possibility of, in effect, constructing an endoscope within a patient. To achieve this, a number of catheters, each providing one or more of the utilities normally provided a conventional endoscope, are successively passed over one or more of the guide wires, so that result is an assemblage of these various elements within the patient. A particular advantage of proceeding in this way is that the force required to advance each of the individual catheters is substantially less than that required to advance a complete conventional endoscope (e.g. a colonoscope or an enteroscope), since the latter is much stiffer and has much greater mass. It is therefore easier for the physician, and less uncomfortable for the patient, and is less likely to cause injury to the patient. Also, since the endoscope is then assembled element by element, the endoscope can have those facilities which are required for the particular patient, and, only those facilities, so that the endoscope is tailored to the requirements of the medical procedure being carried out. It will be understood that, for the purpose of allowing ***in situ*** assembly of a catheter, the guide wire structure should preferably comprise more than two guide wires, for example three or four guide wires.

Although a structure having more than two guide wires is particularly useful for the purpose discussed above of assembling an endoscope ***in situ,*** it may also have value in relation to the procedure for introducing the guide wire structure into a lumen. This is because the two-guide wire structure shown in Figures 1a to 1c allows curvature in only one plane, so that steering the structure in three dimensions requires the user to twist the structure about its longitudinal axis, for example by using a catheter to which the necessary torque can be applied. However, if more than two guide wires are provided it is possible to curve the structure in any plane; three guide wires are sufficient for this purpose.

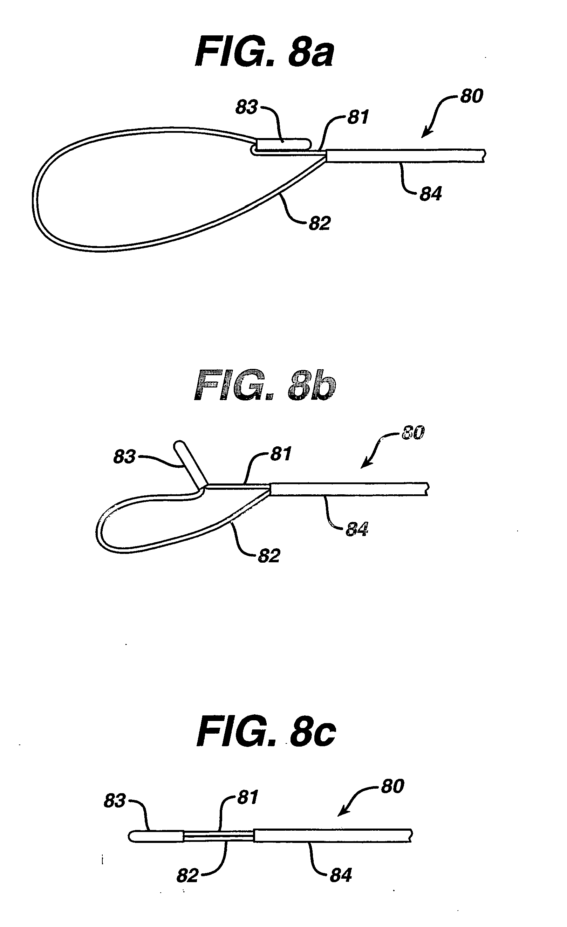

Attention is now directed to Figures 8a to 8c, which illustrate the use of a guide wire structure 80 which comprises two guide wires 81 and 82 connected by a junction portion 83. As can be seen, the junction portion 83 is pivotal about an axis located at the proximal end of the portion 83, so that, as shown in Figure 8a, it can pivot to such an extent that it lies flat along the distal end portion of guide wire 81. This is advantageous in that it makes possible, or makes easier, movement of the portion 83 within a catheter 84, not only where there is no loop present (as in Figure 8c) but also when there is (as shown in Figure 8a). In this connection it is to be understood that the diameter of the catheter 84 would actually be substantially greater than that shown in these Figures. It is also to be understood that instead of being joined by a junction portion 83 of significant length, as illustrated, the guide wires could alternatively be joined by a junction of substantially no length, i.e. the ends of the guide wires could be connected by a junction consisting, at least in substance of just a pivot point.

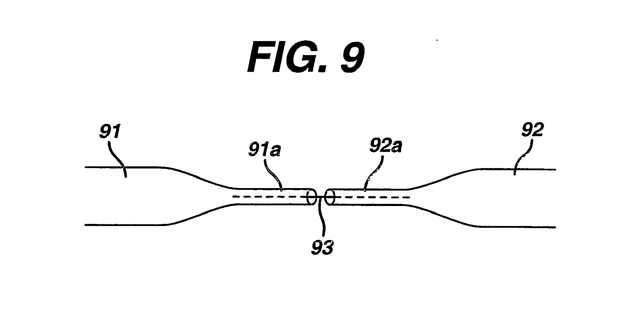

Figure 9 shows yet another guide wire structure in which a similar pivoting action can be achieved. This comprises guide wires 91 and 92, having respective floppy tip portions 91a and 92a connected to one another by a thread or highly flexible wire 93. This thread or wire can be inserted into the portions 91a and 92a, or attached to their surfaces.

Figures 10-13 illustrate a guide wire structure according to the present invention. The guide wire structure includes a continuous, unitary wire having a segment (which can be positioned generally in the middle portion of the wire), which segment has a bending moment of inertia which is lower than the bending moment of inertia of the adjacent wire segments. For instance, the wire can change in cross sectional shape or dimension at a location that is not a terminal end, so as to provide a bending hinge.

The bending moment of inertia for a circular cross-section can be calculated as π r⁴/4, where r is the radius of the cross-section. The bending moment of inertia for a rectangular cross-section can be calculated as bh³/12, where b is the base of the rectangle and h is the height. "Mechanics of Materials", A.C. Ugural, 1991, McGraw Hill is incorporated herein by reference for its disclosure related to bending of cross-sections.

Figure 10 shows an embodiment of guide wire structure of the present invention comprising a continuous, unitary wire 100 that has varying cross sectional area along a portion of its length. In this embodiment, the wire 100 can have a first segment 121 having a generally circular cross section of nominal diameter D101 and a length L101, a second segment 122 having a generally circular cross-section of nominal diameter D102 and a length L102, and a third segment 123 having a generally circular cross-section of nominal diameter D103 and length L103. The wire 100 can also include a tapered transition segment 110 having a conical shape and a length L104 and extending between segment 121 and segment 123, and a tapered transition segment 112 having a length L105 and extending between segment 123 and segment 122.

The reduced diameter D103 of the third segment 123 relative to the diameter D101 and the diameter D102 provides the third segment 123 with a bending moment of inertia which is lower than that of the segments 121 and 122. Accordingly, the wire 100 can bend at the third segment 123 to provide a hinge, which hinge can encompass the length L103 of third segment 123; as well as some or all of the lengths L104 and L105 of segments 110 and 112. In one embodiment, the hinge so formed can be an elastic hinge.

The wire 100 with it's associated hinge can be used in the embodiment as described below, as well as in those methods disclosed with reference to Figures 1-9 above, without the need for attaching or otherwise joining two wires or using different materials.

In one embodiment, the diameters D101 and D102 can be between 0.25 mm (0.010 inch) to 0.89 mm (0.035 inch), and more particularly 0.41 mm (0.016 inch) to 0.51 mm (0.020 inch). The third segment 123 can have a diameter D103 of between 0.13 mm (0.005 inch) and 0.25 mm (0.010 inch), and in one embodiment D103 can be about 0.18 mm (0.007 inch).

Each of L101 and L102 can be at least about 0.9 m (3 feet), and can be between 1.8 m (6 feet) and 3.7m (12 feet). The combined length lengths L101, L102, L103, L104, and L105 can be between 2.1 m (7 feet) and 7.6 m (25 feet). In one embodiment, the lengths L101 and L102 can be about equal, and their combined length can be at least about 6.1 m (20 feet). Length L103 of the third segment can be between 2.64 mm (0.100 inch) to 12.7 mm (0.500 inch), and in one embodiment can be about 7.62 mm (0.300 inch). The length L104 and Length 105 can be about equal, and can each be about 51 mm (2 inches). Modification of the cross section of the wire 100 at a location intermediate the ends may be accomplished by any suitable process, such as by grinding, drawing, or stamping wire 100.

In one embodiment, the reduced cross-section of the third segment 123 can be formed by centerless grinding. A reduced cross-section can be formed using a grinding machine such as a TF-9CPG System 2000 Guide Wire Profile Grinder available from Glebar Company of Franklin Lakes, NJ.

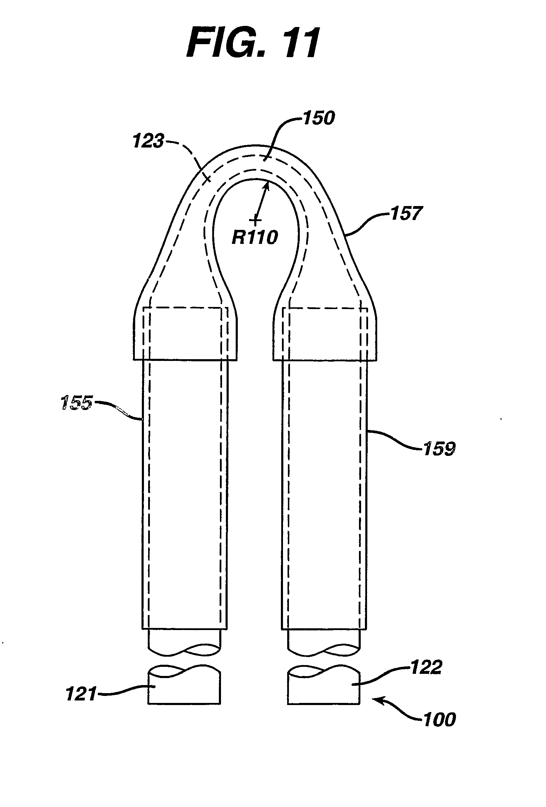

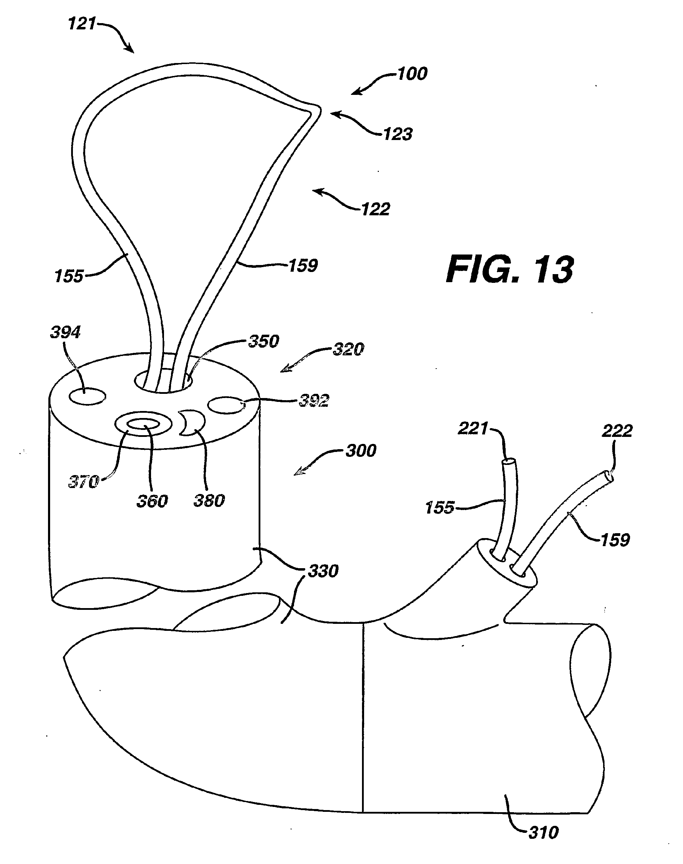

The wire 100 can be enclosed in one or more low friction and/or lubricous sleeves. In Figure 11, the first wire segment 121 is enclosed in a sleeve 155, second wire segment 122 is enclosed in a sleeve 159, and the third segment and the transition segments are enclosed in a sleeve 157. The sleeves 155, 157, and 159 can be formed of a low friction material, such as PTFE or polyester. Indicators are associated with the first and second wire segments 121 and 122 so that the wire segments can be distinguished when viewed through a camera or other optical device associated with an endoscope or other medical device. For instance, the indicators can be,visual, and can employ different colors. In one embodiment, the sleeves 155 and 159 can be provided in different colors and/or with different patterns of markings. In Figure 13, sleeve 155 has a pattern of heavy, diagonally slanted marks, while sleeve 159 has a pattern of lighter, non-slanted markings. The marking colors and/or the background color of the sleeves can be different to distinguish sleeve 155 from sleeve 159. Alternating stripes of different colors can also be used to distinguish sleeve 155 from sleeve 159, and thus segment 121 from 122 as viewed through a visualization device. Sleeve 157 can have yet another color or pattern of colors to provide a visual indication of the location of the third segment 123.

Figure 11 illustrates the wire 100 bent at the third segment 123 to form a narrow wire loop for introduction into a body cavity. In Figure 11, the wire 100 is illustrated with a generally U-shaped bend 150 with a radius R110 so that the wire does not kink upon placement through a colonoscope working channel, does not form a sharp point that can damage tissue upon placement in a body lumen, and preferably does not substantially plastically deform. In one embodiment, the radius R110 can be about 0.75mm to about 1.5mm, and more particularly about 1mm.

Suitable biocompatible materials from which such a wire can be constructed include those mentioned from which the wires of Figures 1-9 can be formed, including without limitation a superelastic material such as nitinol. Other materials, such as steel and alloys can also be used. One suitable material from which wire 100 can be formed is Nitinol NDC SE508 available from Nitinol Devices and Components, a Johnson & Johnson Company of Fremont, CA.

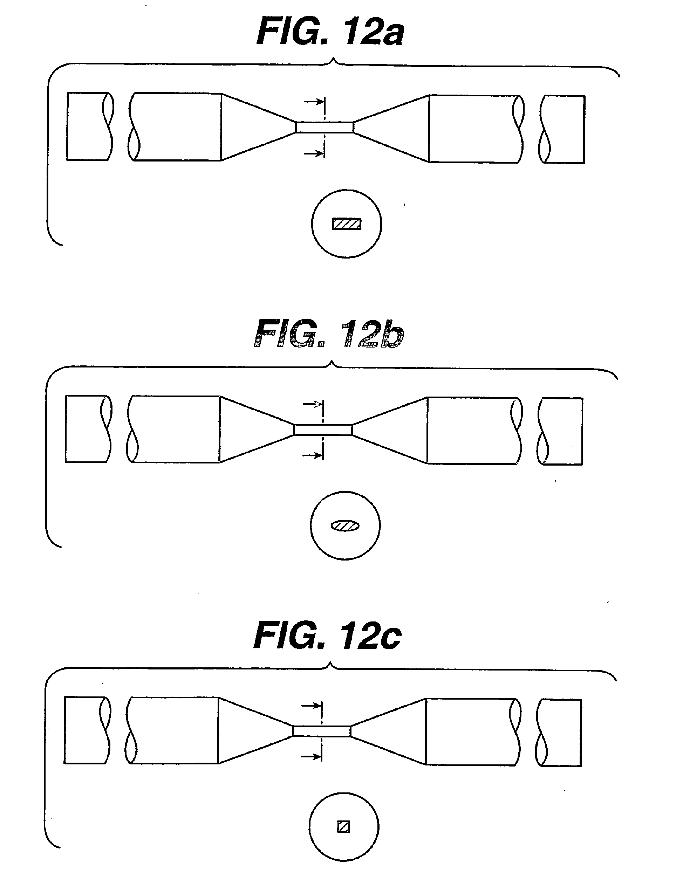

Figure 12 illustrates alternative embodiments in which the cross-section of the narrowed length 103 is not round. Different cross sectional shapes may be formed, such as changing a round wire to a flat rectangular cross section in Figure 12a, an oval cross section in Figure 12b, or a square cross section in Figure 12c. Other cross-sectional shapes, such as triangular, hexangonal, or other polygonal shapes may be employed. Preferential bending planes of certain cross sectional shapes can be used for the purposes of directing the U-loop of the wire. For instance, a rectangular, oval, or triangular cross-section can be employed to direct bending about a particular axis.

The guide wire structure with wire 100 of the present invention can be used in place of the wire configurations shown in Figures 1-9, as well as with the device illustrated in US Patent US 2004/111020 A1. Figure 13 is a schematic illustration of the guide wire structure with wire 100 in use with a medical device 300. Generally, medical device 300 can be a flexible endoscope, such as a flexible colonosocope, or a device such as is shown in the above referenced patent application.

Medical device 300 can include a handle 310, which is positioned outside a patient, an elongate flexible body portion 330, and a distal end 320 which can be positioned in a patient, such as in a patient's GI tract, with distal end 320 sized and shaped to be advanced in the GI tract. The medical device can also include a working channel 350 extending through the body portion 330 and opening at the distal end 320 of the device 300, a camera 360, light source 370, a camera lens wash nozzle/irrigation nozzle 380, a light source 392, and a light source 394. Suction can be provided through working channel 350, if desired.

The guide wire with wire 100 of the present invention can be positioned within the working channel 350 such that the U shaped bend in third segment 123 is positioned in the patient's body, and the ends of the guide wire extend through an access opening of the handle 310. In figure 13, the ends of the guide wire are indicated by numeral 221 (associated with first segment 121) and numeral 222 (associated with second segment 122).

The guide wire with wire 100 can be used generally as shown in Figures 3A-3C to advance a device into a body lumen, such as the GI tract. In Figure 13, after the U shaped bend in the third segment 123 has been advanced through the working channel 350, the first segment 121 is advanced through the working channel relative to second segment 122, so that the first segment 121 takes on a curvature having a radius of curvature greater than Radius of curvature R110, as shown in Figure 13. To advance the distal end 320 further into the patient, the operator can pull proximally on end 222 to move third segment 123 back into the working channel 350, thereby leaving the relatively large radius of curvature loop in first segment 121 in the body lumen and extending from the distal end of the device 300. End 222 can then be held fixed, and end 221 can be advanced distally toward handle 310 so that additional length of the first segment 121 is advanced distally out of working channel 350, thereby advancing the relatively large radius of curvature loop distally in the GI tract. Then, while holding end 222 stationary with respect to handle 310, end 221 can be pulled in tension (proximally) while simultaneously pushing (distally) the elongate body portion 330 distally along wire segments 121 and 122 in working channel 350, so that the distal end 350 moves forward (distally) into the GI tract. Accordingly, the wire segments 121 and 122 serve as a track upon which the distal end 350 of device 300 can be advanced.

While the present invention has been illustrated by description of several embodiments, it is not the intention of the applicant to restrict or limit the scope of the appended claims to such detail. Numerous other variations, changes, and substitutions will occur to those skilled in the art without departing from the scope of the invention. Moreover, the structure of each element associated with the present invention can be alternatively described as a means for providing the function performed by the element. It will be understood that the foregoing description is provided by way of example, and that other modifications may occur to those skilled in the art without departing from the scope of the appended Claims.

## Claims

1. A guide wire structure for insertion into an interior space defined by a wall, the guide wire comprising a continuous, unitary wire (100) comprising a first segment (121), a second segment (122), and a third segment (123) disposed intermediate the first and second segments, wherein the third segment has a bending moment of inertia less than a bending moment of inertia of the first segment and less than a bending moment of inertia of the second segment, **characterized by** comprising a first sleeve (155) encircling the first segment and a second sleeve (159) encircling the second segment, wherein the first and second sleeves are visually distinguishable.

2. The guide wire structure of Claim 1 wherein the third segment (123) has a cross-sectional area less than the cross sectional areas of the first segmet (121) and the second segment (122).

3. The guide wire structure of Claim 1 wherein at least one of the first, second, and third segments (121, 122, 123) have circular cross sections.

4. The guide wire structure of Claim I wherein at least one of the first, second and third segments (121, 122, 123) have non-circular cross-sections.

5. The guide wire structure of Claim 1 wherein the wire is formed of Nitinol.

6. The guide wire structure of Claim 1 wherein the combined length of the first segment, the second segment, and the third segment is at least 2.1 m (7 feet).

7. The guide wire structure of Claim 1 wherein the combined length of the first segment, the second segment, and the third segment is between 2.1 m (7 feet) and 7.6 m (25 feet).

8. The guide wire structure of Claim 1 wherein the combined length of the first segment, the second segment, and the third segment is at least 6.1 m (20 feet).

9. The guide wire structure of Claim 1 wherein the first segment has a length of at least 1.8 m (6 feet), and a generally circular cross-section having a diameter of between 0.28 mm (0.011 inch) to 0.89 mm (0.035 inch).

10. The guide wire structure of Claim 9 wherein the third segment has a diameter of between 0.13 mm (0.005 inch) and 0.25 mm (0.010 inch).

11. The guide wire structure of Claim 1 wherein the first segment (121) has a length of at least 1.8 m (6 feet), wherein the first segment has maximum cross-sectional dimension of no more than 0.89 mm (0.035 inch), and wherein the third segment (123) has a maximum cross-sectional dimension of no more than 0.25 mm (0.010 inch).

12. The guide wire structure of Claim 1 wherein the third segment (123) is bent.

13. The guide wire structure of Claim 1 wherein the third segment (123) provides an elastic hinge.

14. The guide wire structure of any preceding claim, wherein comprising:
the first segment (121) is of a generally constant diameter;
the second segment (122) is of generally constant diameter;
the third segment (123) has a generally constant diameter less than that of the first and
second segment diameters;
and further comprising a tapered segment (110) of decreasing diameter extending from the first segment to the
third segment; and
a tapered segment (112) of decreasing diameter extending from the second segment to the third segment.

## Patentansprüche

1. Führungsdrahtstruktur zum Einführen in einen Innenraum, der durch eine Wand definiert ist, wobei der Führungsdraht einen durchgängigen einheitlichen Draht (100) umfasst mit einem ersten Segment (121), einem zweiten Segment (122) und einem dritten Segment (123), welches zwischen dem ersten und dem zweiten Segment angeordnet ist, wobei das dritte Segment ein Biegeträgheitsmoment aufweist, das kleiner als ein Biegeträgheitsmoment des ersten Segments und kleiner als ein Biegeträgheitsmoments des zweiten Segments ist, **gekennzeichnet durch** eine erste Hülse (155), welche das erste Segment umgibt, und eine zweite Hülse (159), welche das zweite Segment umgibt, wobei die erste und zweite Hülse visuell unterscheidbar sind.

2. Führungsdrahtstruktur nach Anspruch 1, wobei das dritte Segment (123) eine Querschnittsfläche aufweist, die kleiner ist als die Querschnittsflächen des ersten Segments (121) und des zweiten Segments (122).

3. Führungsdrahtstruktur nach Anspruch 1, wobei das erste, zweite und/oder dritte Segment (121, 122, 123) kreisförmige Querschnitte aufweisen.

4. Führungsdrahtstruktur nach Anspruch 1, wobei das erste, zweite und/oder dritte Segment (121, 122, 123) nicht kreisförmige Querschnitte aufweisen.

5. Führungsdrahtstruktur nach Anspruch 1, wobei der Draht aus Nitinol gebildet ist.

6. Führungsdrahtstruktur nach Anspruch 1, wobei die zusammengefasste Länge des ersten Segments, des zweiten Segments und des dritten Segments wenigstens 2,1 m (7 ft.) beträgt.

7. Führungsdrahtstruktur nach Anspruch 1, wobei die zusammengefasste Länge des ersten Segments, des zweiten Segments und des dritten Segments zwischen 2,1 m (7 ft.) und 7,6 m (25 ft.) beträgt.

8. Führungsdrahtstruktur nach Anspruch 1, wobei die zusammengefasste Länge des ersten Segments, des zweiten Segments und des dritten Segments wenigstens 6,1 m (20 ft.) beträgt.

9. Führungsdrahtstruktur nach Anspruch 1, wobei das erste Segment eine Länge von wenigstens 1,8 m (6 ft.) aufweist und einen allgemein kreisförmigen Querschnitt mit einem Durchmesser zwischen 0,28 mm (0,011 Zoll) und 0,89 mm (0,035 Zoll) aufweist.

10. Führungsdrahtstruktur nach Anspruch 9, wobei das dritte Segment einen Durchmesser zwischen 0,13 mm (0,005 Zoll) und 0,25 mm (0,010 Zoll) aufweist.

11. Führungsdrahtstruktur nach Anspruch 1, wobei das erste Segment (121) eine Länge von wenigstens 1,8 m (6 ft.) beträgt und wobei das erste Segment einen maximalen Querschnittsdurchmesser von nicht mehr als 0,89 mm (0,035 Zoll) aufweist und wobei das dritte Segment (123) einen maximalen Querschnittsdurchmesser von nicht mehr als 0,25 mm (0,010 Zoll) aufweist.

12. Führungsdrahtstruktur nach Anspruch 1, wobei das dritte Segment (123) gebogen ist.

13. Führungsdrahtstruktur nach Anspruch 1, wobei das dritte Segment (123) ein elastisches Gelenk zur Verfügung stellt.

14. Führungsdrahtstruktur nach einem der vorhergehenden Ansprüche, wobei das erste Segment (121) einen im Wesentlichen konstanten Durchmesser aufweist; das zweite Segment (122) einen im Wesentlichen konstanten Durchmesser aufweist; und weiterhin Folgendes umfasst:
ein sich verjüngendes Segment (110) mit abnehmenden Durchmesser, das sich von dem ersten Segment zu dem dritten Segment erstreckt; und
ein sich verjüngendes Segment (112) mit abnehmendem Durchmesser, das sich von dem zweiten Segment zu dem dritten Segment erstreckt.

## Revendications

1. Structure de fil guide destinée à être insérée dans un espace intérieur défini par une paroi, le fil guide comprenant un fil unitaire continu (100) comprenant un premier segment (121), un deuxième segment (122), et un troisième segment (123) disposé de façon intermédiaire par rapport aux premier et deuxième segments, dans laquelle le troisième segment possède un moment de flexion d'inertie inférieur à un moment de flexion d'inertie du premier segment et inférieur à un moment de flexion d'inertie du deuxième segment, **caractérisée en ce qu'**elle comprend un premier manchon (155) encerclant le premier segment et un second manchon (154) encerclant le deuxième segment, dans laquelle les premier et second manchons sont visuellement distinguables.

2. Structure de fil guide selon la revendication 1, dans laquelle le troisième segment (123) possède une superficie de section transversale inférieure aux superficies de section transversale du premier segment (121) et du deuxième segment (122).

3. Structure de fil guide selon la revendication 1, dans laquelle au moins un des premier, deuxième et troisième segments (121, 122, 123) possèdent des sections transversales circulaires.

4. Structure de fil guide selon la revendication 1, dans laquelle au moins un des premier, deuxième et troisième segments (121, 122, 123) possèdent des sections transversales non circulaires.

5. Structure de fil guide selon la revendication 1, dans laquelle le fil est formé de Nitinol.

6. Structure de fil guide selon la revendication 1, dans laquelle la longueur combinée du premier segment, du deuxième segment et du troisième segment est au moins 2,1 m (7 pieds).

7. Structure de fil guide selon la revendication 1, dans laquelle la longueur combinée du premier segment, du deuxième segment et du troisième segment est entre 2,1 m (7 pieds) et 7,6 m (25 pieds).

8. Structure de fil guide selon la revendication 1, dans laquelle la longueur combinée du premier segment, du deuxième segment et du troisième segment est au moins 6,1 m (20 pieds).

9. Structure de fil guide selon la revendication 1, dans laquelle le premier segment possède une longueur d' au moins 1,8 m (6 pieds), et une section transversale généralement circulaire possédant un diamètre d'entre 0,28 mm (0,011 pouce) et 0,89 mm (0,035 pouce).

10. Structure de fil guide selon la revendication 9, dans laquelle le troisième segment possède un diamètre d'entre 0,13 mm (0,005 pouce) et 0,25 mm (0,010 pouce).

11. Structure de fil guide selon la revendication 1, dans laquelle le premier segment (121) possède une longueur d'au moins 1,8 m (6 pieds), dans laquelle le premier segment possède une dimension de section transversale maximum non supérieure à 0,89 mm (0,035 pouce), et dans laquelle le troisième segment (123) possède une dimension de section transversale maximum non supérieure à 0,25 mm (0,010 pouce).

12. Structure de fil guide selon la revendication 1, dans laquelle le troisième segment (123) est fléchi.

13. Structure de fil guide selon la revendication 1, dans laquelle le troisième segment (123) fournit une articulation élastique.

14. Structure de fil guide selon une quelconque revendication précédente, comprenant :
✔ le premier segment (121) est d'un diamètre généralement constant ;
✔ le second (122) est d'un diamètre généralement constant ;
✔ le troisième segment (123) possède un diamètre généralement constant inférieur à celui des diamètres des premier et deuxième segments ;
✔ et comprenant en outre un segment tronconique (110) de diamètre diminuant s'étendant du premier segment au troisième segment ; et
✔ un segment tronconique (112) de diamètre diminuant s'étendant du deuxième segment au troisième segment.
